# EUROPÄISCHE PATENTSCHRIFT

(11) **EP 1 091 740 B1**
(45) Veröffentlichungstag und Bekanntmachung des Hinweises auf die Patenterteilung: **20.10.2004**
(21) Anmeldenummer: 99926343.7
(22) Anmeldetag: 20.05.1999
(51) Int. Cl.: A61K 31/53, A61K 47/32

(54) **HALBFESTE WÄSSRIGE ZUBEREITUNGEN FÜR ORALE APPLIKATION VON TOLTRAZURIL-SULFON**
SEMI-SOLID AQUEOUS PREPARATIONS FOR ORAL APPLICATIONS OF TOLTRAZURIL SULPHONE
PREPARATIONS AQUEUSES SEMI-SOLIDES DE SULFONE DE TOLTRAZURILE POUR APPLICATION ORALE

(30) Priorität: 02.06.1998 DE 19824483
(43) Veröffentlichungstag der Anmeldung: 18.04.2001
(73) Patentinhaber: Bayer HealthCare AG, 51368 Leverkusen (DE)
(72) Erfinder: KUHN, Matthias, D-42115 Wuppertal (DE); ROHDE, Bettina, D-51519 Odenthal (DE); SCHNABEL, Helmut, D-51519 Odenthal (DE); MUNDT, Hans-Christian, D-40699 Erkrath (DE)
(86) Internationale Anmeldenummer: PCT/EP1999/003462
(87) Internationale Veröffentlichungsnummer: WO 1999/062519

(56) Entgegenhaltungen:
- EP-A- 0 116 175
- WO-A-98/43644
- DE-A- 2 718 799
- DE-A- 19 519 821

## Beschreibung

Die vorliegende Erfindung betrifft halbfeste wäßrige Zubereitungen für die orale Applikation, welche als wirksamen Bestandteil 1-Methyl-3-[4-[(trifluoromethyl)sulfonyl]phenoxy]-m-tolyl]-s-triazine-2,4,6 (1H, 3H, 5H)-trion (=Toltrazuril-Sulfon) enthalten.

Toltrazuril-Sulfon wird als Wirkstoff in Mitteln gegen Coccidiosen und ähnlichen Erkrankungen bei Tieren verwendet (US-P 4 219 552, DE-P 2 718 799).

Solche Mittel sind üblicherweise Lösungen der Wirkstoffe, die nach Verdünnen mit Wasser über das Trinkwasser der Tiere verabreicht werden (EP-A 116 175). Solche Mittel sind auch Pulver und Granulate die mit dem Futter der unbehandelten Tiere vermischt werden.

In Fällen in denen der Wirkstoff nur schwer wasserlöslich ist, werden wässrige Wirkstoffsuspensionen unter Verwendung geeigneter Suspendiermittel hergestellt. Dazu wird der Wirkstoff in einen Naßmahlverfahren mikronisiert und mit Suspendiermittel und Wasser vermischt. Aus solchen Suspensionen werden dann unter Zusatz von Verdickungsmitteln halbfeste oder pastenförmige Zubereitungen hergestellt. Mikronisierung von Toltrazuril-Sulfon im Naßmahlverfahren führt zu einem Produkt, das sich nicht weiter verarbeiten läßt. Die Herstellung einer stabilen Suspension von Toltrazuril-Sulfon lässt sich daher mit üblichen Methoden nicht erreichen. Damit war auch die übliche Herstellung einer Paste die Toltrazuril-Sulfon als Wirkstoff enthält mit üblichen Mitteln nicht zu erreichen.

Bei einer Mikronisierung von Toltrazuril-Sulfon im Trockenverfahren muß man Teilchengrößen des Wirkstoffs erwarten, die eine stabile wässrige Suspension des Wirkstoffs nur unter Verwendung sehr hoher Suspensionsmittelkonzentrationen erlauben.

Direkt und ohne weitere Verdünnung verabreichbare Mitte wie z.B. orale Pasten sollten jedoch mit möglichst wenig Zusatzstoffen hergestellt sein. Eine auf üblichem Weg hergestellte Paste von Toltrazuril-Sulfon hätte jedoch neben Verdickungsmitteln einen hohen Gehalt an Suspendiermittel enthalten.

Die vorliegende Erfindung betrifft eine oral verabreichbare Paste von Toltrazuril-Sulfon, die dadurch gekennzeichnet ist, daß
a) der Wirkstoff in einer Korngröße von 1 · 10⁻⁶ m und einer maximalen Korngröße von 50 · 10⁻⁶ m in einer Konzentration von 0,1 - 20 Gew.-% vorliegt,
b) Polyacrylsäuren mit einem Acrylsäuregehalt von 56 bis 68 Gew.-% und einem Molekulargewicht von ca. 3 · 10⁶, die mit Alkali oder Erdalkalibasen neutralisiert sind, in einer Konzentration von 0,1 - 5 Gew.-% vorliegen,
c) gegebenenfalls Feuchthaltemittel in einer Konzentration von 5 bis 30 Gew.-% vorliegen,
d) gegebenenfalls Konservierungsstoffe in einer Konzentration von 0,01 bis 0,5 Gew.-% vorliegen,
e) und der Rest in 100 Gew.-% mit Wasser aufgefüllt wird.

Der Wirkstoff ist bevorzugt in den erfindungsgemäßen Formulierungen in Gewichtskonzentrationen von 5 Gew.-% bis 20 Gew.-%, besonders bevorzugt von 10 Gew.-% bis 15 Gew.- % enthalten.

Die Polyacrylsäuren werden bevorzugt mit Alkalihydroxid oder -carbonat neutralisiert. Polyacrylsäuren sind in der erfindungsgemäßen Formulierung in Gewichtskonzentrationen von 0,2 % bis 1 %, bevorzugt von 0,5 % enthalten. Diese sind kommerziell erhältlich und in Arzneibüchern z.B. unter dem Handelsnamen Carbomer 934 P bekannt.

Als Konservierungsmitteln werden bevorzugt Para-hydroxybenzoesäureester (Parabene) wie 4-Hydroxybenzoesäuremethylester, 4-Hydroxybenzoesäureethylester oder 4-Hydroxybenzoesäurepropylester. Die Konservierungsmittel können für eine ausreichende Konservierung einzeln oder in Kombination eingesetzt werden. Sie sind üblicherweise in Konzentrationen von 0,01 - 0,5 Gew.-% enthalten.

Optionsweise kann die Formulierung auch Feuchtehaltemittel enthalten wie z.B. Glycerin oder 1,2-Propylenglycol. Feuchtehaltemittel werden in Gewichtskonzentrationen von 5 % bis 30 %, bevorzugt von 10 % bis 20 % eingesetzt.

Der Wirkstoff liegt in einer Korngröße von 1 bis 10 · 10⁻⁶ m, bevorzugt von 1 bis 5 · 10⁻⁶ m vor. Das Maximum der Korngrößen liegt bei 50 · 10⁻⁶ m, bevorzugt bei 30 · 10⁻⁶ m.

Die gewünschte Korngrößenverteilung des Wirkstoffs wird durch Trockenmahlung erhalten.

Dazu werden z.B. 20 kg Wirkstoff pro Stunde in einer flachzylinderischen Luftstrahlmühle unter einem Druck von 5 bis 6 bar mit Preßluft mikronisiert.

Das erfindungsgemäße Mittel wird durch Zusammenmischen der Einzelkomponenten erhalten. Es kann durch Erhöhen oder Erniedrigen des Wasseranteils in seiner Konsistenz verändert werden. Gewünscht ist eine pastöse Konsistenz. Diese erlaubt die orale Verabreichung des Mittels mit geeigneten Applikatoren wie Spritzen, Tuben, Spatel etc.

Besonders geeignet sind die erfindungsgemäßen Mittel zur Bekämpfung von Coccidiosen und ähnlichen Erkrankungen bei zahlreichen Säugetieren wie Equiden (Pferde, Esel, etc.), Wiederkäuern (Rindern, Schafen, Ziegen, Kameliden, etc.), Schweinen, Hunden, Katzen, Kaninchen, Nagetieren oder anderen Säugern. Die Behandlung kann alle Altersgruppen betreffen. Unter Coccidiosen und ähnlichen Erkrankungen sind Infektionen mit Infektionsstadien von Arten verschiedener Gattungen zu verstehen, wie z.B. Eimeria, Isospora, Castoisospora, Sarkocystis, Toxoplasma, Neospora oder Cryptosporidien. Behandelte Tiere können Endwirte oder Zwischenwirte sein. Die resultierenden Erkrankungen sind unterschiedlich (z.B. Durchfallerkrankungen bei vielen Coccidiosen, Störungen des Bewegungsapparates bei EPM, Aborte, etc). Dementsprechend sind die Anwendungsempfehlungen sehr unterschiedlich. Im allgemeinen sind Dosierungen von bis zu 30 mg Wirkstoff je Körpergewicht, einmalig oder wiederholt verabreicht, wirksam.

Die Paste kann auch unter das Futter der Tiere gemischt werden.

### Beispiel 1

Paste zur oralen Applikation oder zum Mischen unter das Futter

### Zusammensetzung:

| | |
|---|---|
| Toltrazuril-Sulfon | 15 g |
| Polyacrylsäure | 0,5 g |
| Natriumhydroxid | 0,1 g |
| 1,2-Propylenglycol | 20 g |
| Propylparaben | 0,02 g |
| Methylparaben | 0,14 g |
| Wasser | q.s. 100 g |

### Herstellung

Die Komponenten werden zusammengerührt. Es entsteht eine halbfeste wässrige Zubereitung, welche in entsprechenden Applikatoren gefüllt werden kann.

## Patentansprüche

1. Oral verabreichbare Paste von Toltrazuril-Sulfon, die **dadurch gekennzeichnet ist, daß**
a) der Wirkstoff in einer Korngröße von 1 - 10 · 10⁻⁶ m und einem Maximum der Korngrößen bei 50 · 10⁻⁶ m in einer Konzentration von 0,1 - 20 Gew.-% vorliegt,
b) Polyacrylsäuren mit einem Acrylsäuregehalt von 56 % - 68 % (Gew.) und einem Molekulargewicht von ca. 3 · 10⁶ neutralisiert, mit Alkalioder Erdalkalibasen in einer Konzentration von 0,1 - 5 Gew.-% vorliegen,
c) gegebenenfalls Feuchthaltemittel in einer Konzentration von 5 bis 30 Gew.-% vorliegen,
d) gegebenenfalls Konservierungsstoffe in einer Konzentration von 0,01 bis 0,5 Gew.-% vorliegen,
e) und der Rest zu 100 Gew.-% mit Wasser aufgefüllt wird.

2. Verfahren zur Herstellung der Pasten gemäß Anspruch 1, **dadurch gekennzeichnet, daß** man den Wirkstoff mikronisiert und mit den übrigen Komponenten vermischt.

## Claims

1. Orally administrable paste of toltrazuril sulphone, **characterized in that**
a) the active compound, having a particle size of 1 - 10 · 10⁻⁶ m and a particle size maximum at 50 · 10⁻⁶ m, is present in a concentration of 0.1 - 20% by weight,
b) polyacrylic acids having an acrylic acid content of 56% - 68% (by weight) and a molecular weight of about 3 · 10⁶ and neutralized with alkali metal or alkaline earth metal bases, are present in a concentration of 0.1 - 5% by weight,
c) optionally, moisturizers are present in a concentration of from 5 to 30% by weight,
d) optionally, preservatives are present in a concentration of from 0.01 to 0.5% by weight,
e) and the remainder to 100% by weight is made up with water.

2. Process for preparing the pastes according to Claim 1, **characterized in that** the active compound is micronized and mixed with the other components.

## Revendications

1. Pâte de toltrazuril-sulfone, adrninistrable par voie orale, qui est **caractérisée en ce que** :
a) la substance active est présente à une concentration de 0,1 à 20 % en poids avec un diamètre de grains de 1 - 10 · 10⁻⁶ m et un maximum des diamètres de grains d'environ 50 · 10⁻⁶ m,
b) des polymères d'acide acrylique ayant une teneur en acide acrylique de 56 % à 68 % (en poids) et un poids moléculaire d'environ 3·10⁶, neutralisés avec des bases alcalines ou alcaline-terreuses, sont présents à une concentration de 0,1 à 5 % en poids,
c) le cas échéant, des agents retenant l'humidité sont présents à une concentration de 5 à 30 % en poids,
d) le cas échéant, des agents conservateurs sont présents à une concentration de 0,01 à 0,5 % en poids,
e) et le reste est complété à 100 % en poids avec de l'eau.

2. Procédé de production des pâtes suivant la revendication 1, **caractérisé en ce qu'**on micronise la substance active et on la mélange avec les autres composants.
